# EUROPÄISCHE PATENTSCHRIFT

(11) **EP 0 730 567 B1**
(45) Veröffentlichungstag und Bekanntmachung des Hinweises auf die Patenterteilung: **28.10.1998**
(21) Anmeldenummer: 95900766.7
(22) Anmeldetag: 21.11.1994
(51) Int. Cl.: C07C 2/10, B01J 23/78, C07C 11/02

(54) **VERFAHREN ZUR OLIGOMERISIERUNG VON OLEFINEN ZU HOCHLINEAREN OLIGOMEREN UND KATALYSATOREN DAFÜR**
OLIGOMERIZATION PROCESS AND REQUISITE CATALYSTS TO PRODUCE HIGHLY LINEAR OLIGOMERS FROM OLEFINS
PROCEDE D'OLIGOMERISATION D'OLEFINES PERMETTANT D'OBTENIR DES OLIGOMERES A HAUTE LINEARITE, ET CATALYSEURS UTILISES A CET EFFET

(30) Priorität: 22.11.1993 DE 4339713
(43) Veröffentlichungstag der Anmeldung: 11.09.1996
(73) Patentinhaber: BASF AKTIENGESELLSCHAFT, 67056 Ludwigshafen (DE)
(72) Erfinder: VICARI, Maximilian, D-67141 Neuhofen (DE); POLANEK, Peter, D-69469 Weinheim (DE)
(86) Internationale Anmeldenummer: EP9403838
(87) Internationale Veröffentlichungsnummer: WO9514647

(56) Entgegenhaltungen:
- EP-A- 0 329 305
- DE-A- 2 029 624
- US-A- 3 658 935
- US-A- 5 073 658
- US-A- 5 146 030

## Beschreibung

Die Erfindung betrifft ein Verfahren zur Oligomerisierung von Olefinen mit 2 bis 6 Kohlenstoffatomen und Fällungskatalysatoren dafür.

Olefine mit 2 bis 6 Kohlenstoffatomen und deren Gemische, insbesondere Olefine mit 4 Kohlenstoffen stehen in großen Mengen sowohl aus FCC-Anlagen als auch aus Steamcrackern zur Verfügung. Der jeweilige C₄-Schnitt, d.h. das Gemisch aus Butenen und Butanen eignet sich nach Abtrennung des Isobutens sehr gut zur Herstellung von Oligomeren, insbesonders Oktenen und Dodecenen. Sowohl die Oktene als auch Dodecene können nach Hydroformylierung und nachfolgender Hydrierung zu den entsprechenden Alkoholen z.B. für die Herstellung von Weichmachern Verwendung finden.

Für den Einsatz als Weichmacheralkohol spielt der Verzweigungsgrad für die Eigenschaften des Weichmachers eine ausschlaggebende Rolle. Der Verzweigungsgrad wird durch den ISO-Index beschrieben, der die mittlere Zahl der Methylverzweigungen in der jeweiligen Fraktion angibt.

So tragen z.B. n-Oktene mit 0, Methylheptene mit 1 und Dimethylhexene mit 2 zum ISO-Index einer C₈-Fraktion bei. Je niedriger der ISO-Index ist, um so linearer sind die Moleküle in der jeweiligen Fraktion aufgebaut. Je höher die Linearität, d.h. je niedriger der ISO-Index ist, um so höher sind die Ausbeuten in der Oxierung und um so besser sind die Eigenschaften des damit hergestellten Weichmachers. Ein niedriger ISO-Index z.B. bei Phthalatweichmachern wirkt sich günstig in bezug auf eine niedrige Flüchtigkeit und bessere Kältebruch-Temperatur des mit dem Weichmacher hergestellten Weich-PVC aus. Wenig verzweigte Oligomere aus niedrigen Olefinen sind zugänglich aus Umsetzungen sowohl an homogenen wie auch heterogenen Katalysatoren, die als aktive Komponente überwiegend Nickel enthalten. Es sind aber auch andere katalytisch aktive Metalle, wie Ruthenium (G. Braca, La Chimica e l'Industria, 56 (1974), 110-116), Palladium gemäß US 44 36 946 und Kupfer, Cobalt, Eisen, Chrom und Titan gemäß GB 824 002 beschrieben. Technische Bedeutung haben allerdings nur die nickelhaltigen Katalysatoren erlangt.

DE 28 55 423 offenbart als homogenen Katalysator ein System, das aus dem Nickel-II-Salz der Oktansäure, Ethylaluminiumdichlorid und einer freien Fettsäure besteht. Ein Katalysatorsystem dieser Art wird auch beim einzigen homogen katalysierten Verfahren von technischer Bedeutung zur Olefinoligomerisierung (Y. Chauvin, Chemistry and Industry, 1974, 375-378) eingesetzt.

Homogen katalysierte Verfahren zur Oligomerisierung von Olefinen sind problematisch einerseits wegen der Abtrennung des Katalysators, andererseits wegen der im Vergleich zu einer heterogenkatalytischen Fahrweise wesentlich höheren Katalysatorkosten pro Tonne Produkt. Bei der Abtrennung und Vernichtung des Katalysators, der in geradem Durchgang mit dem Einsatzproduktstrom durch die Anlage gefahren wird, entsteht außerdem schwermetall- und ammoniumhaltiges Abwasser, das entsprechend aufgearbeitet und entsorgt werden muß.

Neben den homogenen Katalysatoren sind auch zahlreiche heterogene Katalysatorsysteme auf Basis Nickel und Silicium beschrieben, die oftmals zusätzlich noch Aluminium enthalten und auf unterschiedlichste Weise hergestellt werden.

US 5 169 824 offenbart beispielsweise die Herstellung eines Nickel-Fällungskatalysators auf Al₂O₃/SiO2. Der Gehalt an NiO ist so abgestimmt, daß eine Einschichtbelegung des Al₂O₃/SiO₂-Trägers, d.h. 0,07 bis 0,12 Gew.-% NiO pro 1 m² Trägeroberfläche erhalten wird.

Mit diesem Katalysator wird, bei für das Einsatzolefin trans-2-Buten überkritischen Bedingungen und unter Zugabe von Dekan als unterkritischem Lösungsmittel ein Umsatz im Autoklaven von 86 %, eine C₈-Selektivität von 53 % und ein ISO-Index von 1,3 in der C₈-Fraktion erhalten.

Nach DD 273 055 wird Nickel und Aluminium auf SiO₂ aufgefällt. Mit einem wasserstoffgesättigten Buten/Butan-Gemisch wird ein Umsatz von 57 % zu Oligomeren mit 91 % C₈-Selektivität und einem ISO-Index von 1,2 erreicht.

Ein Katalysator zur Codimerisierung von Propylen und n-Butenen kann nach DE 20 51 402 durch gemeinsames Ausfällen, ausgehend von einem Kieselsäuresol, einem Nickelsalz und kolloidalem Aluminiumoxid erhalten werden.

Nach EP 202 670 kann ein oligomerisierungsaktiver Katalysator durch Tränken von Aluminiumoxidformkörpern mit Ni-Salz, Calcinieren und Aktivieren durch Imprägnieren mit Aluminiumchlorid und Diethylaluminiumchlorid hergestellt werden.

DE-A 20 29 624 beschreibt Katalysatoren auf Basis von Kieselsäuregel und deren Verwendung in Verfahren zur Oligomerisierung von Olefinen, die - bezogen auf das Gewicht des Kieselsäuregels - 0,1 bis 5 Gew.-% Aluminium, 0,1 bis 5 Gew.-% Alkalimetalle und 0,1 bis 5 Gew.-% Nickel enthalten.

Andere Katalysatoren können erhalten werden, indem man die an der Trägeroberfläche befindlichen positiv geladenen Teilchen, wie Protonen, Alkali- oder Erdalkali-Ionen gegen Nickel-Ionen austauscht. Dabei kommen unterschiedlichste Trägermaterialien zum Einsatz, z.B. gemäß R. Espinoza, Appl. Catal. 31 (1987), S. 259-266, amorphes Aluminiumsilikat, gemäß EP 261 730 Zeolith-Y mit Fe, Cu, La, Ca, Ni oder Co ausgetauscht, gemäß NL 8 500 459 Zeolithe vom ZSM-Typ, gemäß DE 2 347 235 ein X-Zeolith, oder gemäß US 5 134 242 ein Zeolith mit MCM-41-Struktur oder ein nickelhaltiger Zeolith hergestellt aus Cs oder Ba ausgetauschtem CZS-1 oder ultrastabiler hoch Si-haltiger Faujasit nach EP 329 305.

Außer diesen als Beispiele angeführten Katalysatoren sind z.B. Katalysatoren auf Basis halogenfreier Titan- bzw. Zirkonsalze auf Tonmasse gemäß US 5 146 030 und Katalysatoren mit disperser TiO₂-Phase auf SiO₂-einschichtbelegtem Al₂O₃ als Träger gemäß US 5 073 658 beschrieben.

Des weiteren sind gemäß US 5 113 034 Katalysatoren auf Basis NiO/ZrO₂/SO₄/SiO₂ oder TiO₂/SO₄ für die Oligomerisierung von Propen und Butenen beschrieben.

Bedingt durch die hohe Acidität des Trägermaterials werden mit den Katalysatoren, z.B. auf Basis Zeolith, oder supersauren Katalysatoren, wie ZrO₂/SO₄, TiO₂/SO₄, stark verzweigte Oligomere erhalten.

Bei hoher Acidität des Katalysators erfolgt die Oligomerisierung über einen kationischen Mechanismus, der für Butene als Einsatzstoff zwangsweise über das stabilere 2-Butyl-Kation größtenteils zu Dimethylhexenen und Methylheptenen führt.

Für die Herstellung von Weichmachern wird aber - wie schon erläutert - eine möglichst hohe Linearität der Oligomeren, d.h. niedriger ISO-Index (max. 1 - 1,2) gefordert, um hochwertige Weichmacher zu erhalten.

Hochlineare Olefine werden erhalten, wenn die Oligomerisierung über einen koordinativen Mechanismus abläuft. Nur über den koordinativen Mechanismus können n-Oktene erhalten werden, die definitionsgemäß mit dem Faktor O zum ISO-Index der C₈-Fraktion beitragen.

Um die hohe Reaktionswärme, die bei der Oligomerisierung frei wird, wirtschaftlich nutzen zu können, d.h. z.B. die Reaktionswärme für die nachgeschalteten Trennkolonnen zu nutzen, ist es vorteilhaft, die Oligomerisierung oberhalb einer Reaktortemperatur von 160°C und bei überkritischem Druck bezüglich des Buten/Butangemisches durchzuführen, wobei sich z. B. das eingesetzte Buten/Butan-Gemisch im überkritischen Zustand befindet.

Die hohe Reaktortemperatur stellt besondere Anforderungen an den Katalysator, die im wesentlichen in drei Punkten zusammengefaßt werden können:
1. Verhinderung der Bildung von höheren Oligomeren, die an der aktiven Oberfläche des Katalysators als Koks-Vorläufer haften bleiben.
2. Hohe Stabilität der hochdispersen NiO-Phase gegenüber Agglomeration.
3. Hohe Stabilität der aktiven NiO-Phase gegenüber Reduktion.

Dementsprechend wurde ein Verfahren zum Oligomerisieren von unverzweigten C₂- bis C₆-Olefinen zu deren Dimeren, Trimeren und Tetrameren mittels eines Festbettkatalysators, bei erhöhtem Druck, bei Raumtemperatur oder bei erhöhter Temperatur gefunden, das dadurch gekennzeichnet ist, daß man einen Katalysator verwendet, der als aktive Bestandteile, nach Abzug des Glühverlustes nach Temperung bei 900°C, 10 bis 70 Gew.-% Nickeloxid, berechnet als NiO, 5 bis 30 Gew.-% Titandioxid und/oder Zirkoniumdioxid, 0 bis 20 Gew.-% Aluminiumoxid, 20 bis 40 Gew.-% Siliciumdioxid und 0,01 bis 1 Gew.-% eines Alkalimetalloxids enthält, mit der Maßgabe, daß sich die Gehalte an den einzelnen Komponenten im Katalysator zu 100 Gew.-% ergänzen.

Nach einer bevorzugten Ausführungsform führt man die Oligomerisierung unter überkritischen Bedingungen in bezug auf das Einsatzmaterial durch, wobei man vorzugsweise keine zusätzlichen Lösungsmittel verwendet, die sich nicht im überkritischen Zustand befinden.

Erfindungsgemäß bewirkt der Zusatz von festem TiO₂ und/oder ZrO₂, daß die Nickeloxidphase im Katalysator hochdispers und hochstabil im bezug auf Reduktion zu metallischem Nickel vorliegt und so eine hohe Aktivität gewährleistet.

Die Bildung höherer Oligomere kann nach US 5 169 824 durch Einbringen dreiwertiger Metalloxide, wie Al₂O₃ oder Ga₂O₃, die acide Zentren im Katalysator erzeugen, inhibiert werden. Das Metalloxid kann nach dem Stand der Technik sowohl mit dem SiO2-Träger mitgefällt, als auch durch Tränkung in den Katalysatorträger eingebracht werden. Dabei muß, um Oligomere mit möglichst hoher Linearität zu erhalten, die Einbringung und die Menge des Metalloxids so abgestimmt sein, daß einerseits die Bildung großer Oligomeren inhibiert, andererseits die Acidität des Trägers nicht zu groß wird. Hierdurch wird gewährleistet, daß die Reaktion bevorzugt nach einem koordinativen Mechanismus abläuft.

Erfindungsgemäß kann bei gleichzeitigem Fällen der Metallsalzlösung und des Aluminiumsalzes eine minimal notwendige Trägeracidität eingestellt werden, so daß bei hoher Aktivität des Oligomerisierungskatalysators noch hochlineare Oligomere erhalten werden.

Es wurde nun ferner gefunden, daß bei der Herstellung eines Oligomerisierungskatalysators, der die Oxide der Metalle, Nickel, Silicium, gegebenenfalls Aluminium und Titan und/oder Zirkon oder beide letztgenannten Metalloxide enthält, hinsichtlich der gewünschten Eigenschaften besonders vorteilhafte Ergebnisse erzielt werden, wenn man bei der Fällung der Metallsalzlösung mit Alkalicarbonatlösung einerseits die titanhaltige oder zirkonhaltige Komponente als festes Titandioxid bzw. Zirkoniumdioxid oder Zirkoniumhydroxid vorlegt und andererseits die Aluminiumkomponente mit der Metallsalzlösung gleichzeitig mitfällt. Nach der Fällung wird die erhaltene Suspension filtriert, gewaschen, getrocknet und getempert.

Hinsichtlich der Eigenschaften des Oligomerengemisches werden besonders vorteilhafte Ergebnisse erhalten, wenn die Fällung bei einer Temperatur von im allgemeinen 30 - 90°C und einem pH-Wert von im allgemeinen 5 - 9, vorzugsweise von 6,5 - 7,5, und das Tempern bei einer Temperatur von im allgemeinen 350 - 650°C, vorzugsweise von 450 - 550°C, durchgeführt wird. Dabei besteht die Katalysatormasse nach dem Tempern aus 5 - 30 Gew.-% Titandioxid und/oder Zirkoniumdioxid, 0 - 20 Gew.-% Aluminiumoxid, 10 - 70 Gew.-% Nickeloxid, berechnet als NiO, und als Rest Siliciumdioxid.

Das Verfahren zur Herstellung des erfindungsgemäßen Oligomerisierungskatalysators wird z.B. im einzelnen so durchgeführt, daß man Alkaliwasserglas, vorzugsweise Natronwasserglas, in wäßriger Lösung vermischt und mit festem Titandioxid vorlegt und bei im wesentlichen konstantem pH-Wert die Metallsalzlösung, die die entsprechenden Metallsalzmengen von Nickel und Aluminium enthält, mit der Alkalicarbonatlösung vermischt, wobei die Metalle in Form eines Gemisches von Metallhydroxiden und Metallcarbonaten ausfallen. Als Metallsalze verwendet man vorzugsweise die Nitrate, Sulfate oder Acetate der Metalle. Der Metallsalzgehalt der Metallsalzlösungen beträgt im allgemeinen 30 - 40 Gew.-% an Nickelsalz und gegebenenfalls 10 - 15 Gew.-% an Aluminiumsalz. Die Alkalicarbonatlösung ist im allgemeinen 15 - 25, vorzugsweise 18 - 22 gew.-%ig. Die Fällung wird bei einer Temperatur von im allgemeinen 30 - 90°, vorzugsweise 60 - 80°C und einem pH-Wert von 5 - 9, vorzugsweise 6,5 - 7,5, durchgeführt. Die erhaltene Suspension wird filtriert und solange gewaschen, bis keine Anionen der gefällten Metallsalze mehr nachgewiesen werden können. Anschließend wird z.B. bei einer Temperatur von 150°C im Trockenschrank oder einem Sprühtrockner getrocknet. Der getrocknete Filterkuchen wird bei einer Temperatur von im allgemeinen 350 bis 650°C, vorzugsweise 400 bis 600°C, z.B. in einem Muffelofen oder Drehrohr getempert.

Die so erhaltene Katalysatormasse, die beim Tempern bei einer Temperatur von 900°C noch einen Glühverlust von ca. 5 bis 15 Gew.-% besitzt, enthält, nach Abzug des Glühverlustes, 5 bis 30 Gew.-% Titandioxid, vorzugsweise 10 bis 20 Gew.-% Titandioxid, 0 bis 20 Gew.-% Aluminiumoxid, vorzugsweise 0,5 bis 10 Gew.-% Aluminiumoxid, 10 bis 70 Gew.-% Nickeloxid, vorzugsweise 40 bis 60 Gew.-% Nickeloxid, berechnet als NiO, als Hauptbestandteil und als Rest zu 100 Gew.-% Siliciumdioxid. Im allgemeinen beträgt dieser Siliciumdioxid-Gehalt 20 bis 40 Gew.-%. Infolge seiner Herstellungsweise enthält der Katalysator im allgemeinen noch 0,01 bis 1 Gew.-%, vorzugsweise 0,1 bis 0,3 Gew.-% des zur Fällung verwendeten Alkalimetalls, berechnet als Alkalimetalloxid. Die Einhaltung dieses Alkalimetallgehalts wirkt sich vorteilhaft auf die Aktivität und Selektivität des Katalysators aus.

Diese Katalysatormasse wird vor dem Einsatz auf die übliche Weise tablettiert oder extrudiert. Zum Beispiel verpreßt man die Katalysatormasse unter Verwendung eines Tablettierhilfsmittels, vorzugsweise Graphit oder Stearinsäure, zu Tabletten mit z.B. den Dimensionen 3 mm Höhe x 3 mm Durchmesser, 5 x 5 mm, 6 x 3 mm oder Ringen mit z.B. 7 mm Außendurchmesser, 5 mm Höhe und 3 mm Lochdurchmesser. Wenn Stearinsäure als Tablettierhilfsmittel Anwendung findet, ist es vorteilhaft, die Katalysatorformkörper nochmals bei einer Temperatur von 350 bis 650°C, vorzugsweise 400 bis 600°C, z.B. in einem Muffelofen oder Drehrohr zu tempern.

Die auf diese Weise hergestellten Katalysatoren werden vor dem Einsatz zweckmäßigerweise einer Konditionierung im trockenen Stickstoffstrom, z.B. bei Atmosphärendruck und Temperaturen von 150 - 500°C, vorzugsweise 250 bis 450°C unterworfen, um noch enthaltenes Wasser aus dem Katalysator zu entfernen.

Die erfindungsgemäßen Katalysatoren eignen sich hervorragend für die Oligomerisierung von Butenen in Buten/Butan-Gemischen. Für die Oligomerisierung von Butenen mit den erfindungsgemäßen Katalysatoren sind Reaktionstemperaturen von 20 bis 280°C, vorzugsweise oberhalb 160°C, insbesondere von 180 bis 210°C und ein Reaktionsdruck von 10 bis 300 bar, vorzugsweise 20 bis 300 bar, insbesondere 60 bis 80 bar, vorteilhaft. Überraschenderweise haben die erfindungsgemäßen Katalysatoren bei ihrem Einsatz im erfindungsgemäßen Verfahren bezüglich angewandtem Reaktionsdruck und angewandter Reaktionstemperatur zwei optimale Arbeitsbereiche, nämlich a) bei einem Druck von 10 bis 30 bar, insbesondere 15 bis 25 bar und bei 20°C bis 140°C, vorzugsweise 40 bis 120°C und b) bei einem Druck von 60 bis 300 bar, insbesondere 60 bis 80 bar, und bei einer Temperatur von 160 bis 280°C insbesondere 180 bis 210°C. Das Vorliegen zweier optimaler Arbeitsbereiche ermöglicht den Einsatz der erfindungsgemäßen Katalysatoren in mit unter unterschiedlichen Prozeßbedingungen betriebenen Butenoligomerisierungsanlagen. Mit den erfindungsgemäßen Katalysatoren können in hoher Ausbeute hochlineare, dimere und trimere Olefine erhalten werden.

Dabei ist überraschend, daß trotz einer erhöhten Acidität im Katalysator bedingt durch die Dotierung mit Aluminiumoxid, eine hohe Linearität der Dimeren- und Trimerenfraktion erhalten wird.

### Beispiel 1

902,9 g Ni(NO₃)₂ * 6 H₂O (entspricht 225 g NiO) werden in vollentsalztem Wasser auf 3000 ml gelöst (Metallsalzlösung). 2000 ml vollentsalztes Wasser werden in einem Rührgefäß vorgelegt und 60,7 g feinteiliges Titandioxidpulver (entspricht 58,5 g reinem (100 %) TiO₂) sowie 616 g Natronwasserglaslösung (entspricht 166,5 g SiO₂) unter Rühren zugegeben. Der Ansatz wird auf 70°C erwärmt und die Metallsalzlösung wird unter Rühren zugepumpt, bis sich ein pH-Wert von 7,0, gemessen mit einer Glaselektrode, einstellt. Dann wird in kontinuierlichem Strom gleichzeitig die Metallsalzlösung und 20 gew.-%ige Soda-Lösung zugegeben, wobei der mit der Glaselektrode gemessene pH-Wert von 7,0 aufrechterhalten wird. Nach vollständiger Zugabe der Metallsalzlösung wird noch 1 Stunde ohne Zugabe von weiterer Soda-Lösung nachgerührt. Die Fällung wird filtriert und mit vollentsalztem Wasser gewaschen bis die elektrische Leitfähigkeit kleiner 30 µS beträgt. Danach wird der Filterkuchen bei einer Temperatur von 150°C in einem Trockenschrank oder einem Sprühtrockner getrocknet. Das auf diese Weise erhaltene Hydroxidcarbonatgemisch wird nun bei einer Temperatur von 500°C über einen Zeitraum von 4 Stunden getempert. Der so erhaltene Katalysator hat folgende Zusammensetzung: 50 Gew.-% NiO, 36,6 Gew.-% SiO₂, 13 Gew.-% TiO₂ und 0,4 Gew.-% Na₂O. Das Katalysatorpulver wird mit 3 Gew.-% Graphit vermischt und zu 3 x 3-mm-Tabletten verpreßt.

### Vergleichsbeispiel 1 (Katalysator ohne TiO₂)

902,9 g Ni(NO₃)₂ * 6 H₂O (entspricht 225 g NiO) werden in vollentsalztes Wasser auf 3000 ml gelöst (Metallsalzlösung) . 2000 ml vollentsalztes Wasser wird in einem Rührgefäß vorgelegt und 832 g Natronwasserglaslösung (entspricht 225 g SiO₂) unter Rühren zugegeben. Der Ansatz wird auf 70°C erwärmt und die Metallsalzlösung unter Rühren zugepumpt, bis sich ein pH-Wert von 7,0, gemessen mit einer Glaselektrode, einstellt. Dann wird in kontinuierlichem Strom gleichzeitig die Metallsalzlösung und 20 Gew.-%ige Soda-Lösung zugegeben, wobei der mit der Glaselektrode gemessene pH-Wert von 7,0 aufrechterhalten wird. Nach vollständiger Zugabe der Metallsalzlösung wird noch 1 Stunde ohne Zugabe von weiterer Soda-Lösung nachgerührt. Die Füllung wird filtriert und mit vollentsalztem Wasser gewaschen bis die elektrische Leitfähigkeit kleiner 30 ~S beträgt. Danach wird der Filterkuchen bei einer Temperatur von 150°C in einem Trockenschrank oder einem Sprühtrockner getrocknet. Das auf diese Weise erhaltene Hydroxid- carbonatgemisch wird nur bei einer Temperatur von 500°C über einen Zeitraum von 4 Stunden getempert. Der so erhaltene Katalysator hat folgende Zusammensetzung: 50 Gew.-% NiO, 49,5 Gew.-% SiO₂ und 0,5 Gew.-% Na₂O. Das Katalysatorpulver wird mit 3 Gew.-% Graphit vermischt und zu 3 x 3-mm-Tabletten verpreßt.

### Beispiel 2

Man verfährt wie in Beispiel 1 beschrieben, verwendet jedoch eine Lösung aus
902,9 g Ni(NO₃)₂ * 6 H₂O (=A 225 g NiO) und
348,5 g Al(NO₃)₃ * 9 H₂O (=~ 45 g Al₂O₃), sowie
76,5 g feinteiliges Titandioxidpulver (=A 58,5 g TiO₂ 100 %) und
450 g Natronwasserglaslösung (=~ 121,5 g SiO₂) zur Fällung
und arbeitet wie dort beschrieben auf.

Der so erhaltene Katalysator hat folgende Zusammensetzung: 50 Gew.-% NiO, 26,7 Gew.-% SiO₂, 10 Gew.-% Al₂O₃, 13 Gew.-% TiO₂ und 0,3 Gew.-% Na₂O. Das Katalysatorpulver wird mit 3 Gew.-% Graphit vermischt und zu 3 x 3-mm-Tabletten verpreßt.

### Vergleichsbeispiel 2

### (Verwendung festen Aluminiumoxids anstelle einer Mitfällung des Aluminiumoxids)

Man verfährt wie in Vergleichsbeispiel 1, verwendet jedoch in der Vorlage
76,5 g feinteiliges Titandioxidpulver (≙ 58,5 g TiO₂; 100 %),
62,8 g feinteiliges Böhmitpulver (≙ 45 g Al₂O₃; 100 %) sowie
450 g Natronwasserglaslösung (≙ 121,5 g SiO₂).

Der so erhaltene Katalysator hat folgende Zusammensetzung:
50 Gew.-% NiO,
26,8 Gew.-% SiO₂,
10 Gew.-% Al₂O₃ und
13 Gew.-% TiO₂,
0,2 Gew.-% Na₂O.

Das Katalysatorpulver wird mit 3 Gew.-% Graphit vermischt und zu 3 x 3-mm-Tabletten verpreßt.

### Beispiel 3

Die folgenden Umsetzungen wurden kontinuierlich unter Verwendung eines Festbettreaktors unter im Vergleich zum Eigendruck der Butene bzw. Butane erhöhtem Druck durchgeführt. Der Druck wird über die Reaktoreinsatzpumpe vor dem Reaktor erzeugt und über eine Druckhaltung nach dem Reaktor und nach der Reaktionsproduktkühlung entsprechend geregelt. Folgende Bedingungen wurden bei den Vergleichsversuchen konstant gehalten:

Einsatzproduktgemisch:

| | |
|---|---|
| n-, i-Butan | 33 Gew.-% |
| 1-Buten | 11 Gew.-% |
| trans-2-Buten | 35 Gew.-% |
| cis-2-Buten | 20 Gew.-% |
| i-Buten | 1 Gew.-% |

Katalysatorbelastung: 0,5 kg/l * h

Im Folgenden sind die Ergebnisse der Umsetzung mit dem erfindungsgemäßen Katalysator auf Basis NiO/SiO₂/TiO₂ und dem Vergleichskatalysator auf Basis NiO/SiO₂ gemäß Beispiel 1 und Vergleichsbeispiel 1 gegenübergestellt, um den Einfluß der 5 TiO₂-Dotierung zu zeigen.

| | | |
|---|---|---|
| Druck [bar] | 70 | |
| Temperatur [°C] | 190 | |
| Katalysator | gemäß Beispiel 1 | gemäß Vergleichsbeispiel 1 |
| Gesamt Buten-Umsatz [Gew.-%] | 53 | 41 |
| C₈ [Gew.-%) | 73 | 78 |
| C₁₂ [Gew.-%) | 20 | 17 |
| C₁₆+* [Gew.-%) | 7 | 5 |

| C₈-Fraktion: | | |
|---|---|---|
| Methylheptene | 65 | 67 |
| Dimethylhexene | 7 | 9 |
| Trimethylpentene | 1 | 3 |
| n-Oktene | 24 | 21 |
| ISO-Index | 0,85 | 0,94 |

Bei höherem Umsatz wird ein Oligomerengemisch mit geringerem ISO-Index, also geringer Verzweigung, erhalten.
* C₁₆+: Oligomerisierungsprodukte mit 16 und mehr Kohlenstoffatomen.

### Beispiel 4

Der erfindungsgemäße-Katalysator gemäß Beispiel 1 auf Basis 50 Gew.-% NiO/37 Gew.-% SiO₂/13 Gew.-% TiO₂ liefert die folgenden Ergebnisse in flüssiger Phase und im überkritischen Zustand im Bezug auf die eingesetzten Butene und Butane.

| | | |
|---|---|---|
| Druck [bar) | 30 | 70 |
| Temperatur [°C] | 80 | 190 |
| Gesamt Buten-Umsatz | 54 | 53 [Gew.-%) |
| C₈ [Gew.-%) | 72 | 73 |
| C12 [Gew.-%) | 20 | 20 |
| C₁₆+ [Gew.-%) | 8 | 7 |
| C₈-Fraktion: | | |
| Methylheptene | 73 | 65 |
| Dimethylhexene | 16 | 7 |
| Trimethylpentene | - | 1 |
| In-Oktene | 11 | 24 |
| ISO-Index | 1,1 | 1,1 |

Bei nahezu gleichem Umsatz und gleichem ISO-Index wird die doppelte Menge der erwünschten n-Octene erhalten.

### Beispiel 5

Man verfährt wie in Beispiel 3 beschrieben unter Verwendung der in Beispiel 2 und Vergleichsbeispiel 2 beschriebenen Katalysatoren und erhält folgendes Ergebnis:

| | | |
|---|---|---|
| Druck [bar) | 70 | |
| Temperatur [°C) | 190 | |
| Katalysator | gemäß Beispiel 2 | gemäß Vergleichsbeispiel 2 |
| Gesamt Buten-Umsatz [Gew.-%] | 78 | 55 |
| C₈ [Gew.-%) | 62 | 70 |
| C₁₂ [Gew.-%] | 25 | 21 |
| C₁₆+ [Gew.-%] | 13 | 9 |
| C₈-Fraktion: | | |
| Methylheptene | 68 | 64 |
| Dimethylhexene | 9 | 9 |
| Trimethylpentene | 2 | 3 |
| n-Oktene | 21 | 24 |
| ISO-Index | 0,92 | 0,91 |

Bei gleichem ISO-Index erhält man mit dem erfindungsgemäßen Katalysator ein deutlich höheren Umsatz.

## Patentansprüche

1. Verfahren zum Oligomerisieren von unverzweigten C₂- bis C₆-Olefinen zu deren Dimeren, Trimeren und Tetrameren mittels eines Festbettkatalysators, bei erhöhtem Druck, bei Raumtemperatur oder bei erhöhter Temperatur, dadurch gekennzeichnet, daß man einen Katalysator verwendet, der als aktive Bestandteile, nach Abzug des Glühverlustes nach Temperung bei 900°C, 10 bis 70 Gew.-% Nickeloxid, berechnet als NiO, 5 bis 30 Gew.-% Titandioxid und/oder Zirkoniumdioxid, 0 bis 20 Gew.-% Aluminiumoxid, 20 bis 40 Gew.-% Siliciumdioxid und 0,01 bis 1 Gew.-% eines Alkalimetalloxids enthält, mit der Maßgabe, daß sich die Gehalte an den einzelnen Komponenten im Katalysator zu 100 Gew.-% ergänzen.

2. Verfahren nach Anspruch 1, dadurch gekennzeichnet, daß man einen Katalysator gemäß Anspruch 7 verwendet.

3. Verfahren gemäß Anspruch 1, dadurch gekennzeichnet, daß man die Oligomerisierung in der Reaktionszone bei überkritischer Temperatur und überkritischem Druck der eingesetzten Olefine durchführt und keine zusätzlichen Lösungsmittel verwendet, die sich in der Reaktionszone nicht im überkritischen Zustand befinden.

4. Verfahren gemäß Anspruch 1, dadurch gekennzeichnet, daß man C₄-Olefine oligomerisiert und die Oligomerisierung bei Temperaturen von 20 bis 280°C und bei einem Druck von 10 bis 300 bar durchführt.

5. Verfahren nach Anspruch 1, dadurch gekennzeichnet, daß man C₄-Olefine oligomerisiert und die Oligomerisierung bei einem Druck von 10 bis 30 bar und bei einer Temperatur von 20 bis 140°C durchführt.

6. Verfahren nach Anspruch 1, dadurch gekennzeichnet, daß man C₄-Olefine oligomerisiert und die Oligomerisierung bei einem Druck von 60 bis 300 bar und bei einer Temperatur von 160 bis 280°C durchführt.

7. Katalysatoren für die Oligomerisierung von Olefinen mit 2 bis 6 Kohlenstoffatomen gemäß Anspruch 1, bestehend im wesentlichen aus Nickeloxid, Siliciumdioxid, Titandioxid und/oder Zirkoniumdioxid sowie gegebenenfalls Aluminiumoxid mit einem Gehalt, nach Abzug des Glühverlustes nach Temperung bei 900°C, an Nickeloxid, berechnet als NiO, von 10 bis 70 Gew.-%, 5 bis 30 Gew.-% Titandioxid und/oder Zirkoniumdioxid, 0 bis 20 Gew.-% Aluminiumoxid 20 bis 40 Gew.-% Siliciumdioxid und 0,01 bis 1 Gew.-% eines Alkalimetalloxids, mit der Maßgabe, daß sich die Anteile der einzelnen Komponenten zu 100 Gew.-% ergänzen, erhältlich durch Fällung einer Aluminium-freien oder ein gelöstes Aluminiumsalz enthaltenden Nickelsalzlösung bei einem pH-Wert von 5 bis 9 durch Zugabe dieser Nickelsalzlösung zu einer Alkaliwasserglaslösung, die festes Titandioxid und/oder Zirkoniumdioxid enthält, Trocknung und Temperung des erhaltenen Präzipitats bei 350 bis 650°C.

8. Katalysatoren gemäß Anspruch 7, erhältlich durch Fällung bei einer Temperatur von 30 bis 90°C.

## Claims

1. A process for the oligomerization of straight-chain C₂-C₆-olefins to their dimers, trimers and tetramers by means of a fixed-bed catalyst, at superatmospheric pressure and at room temperature or elevated temperatures, wherein the catalyst used is one which contains, as active components, after deduction of the loss on ignition following heating at 900°C, from 10 to 70% by weight of nickel oxide, calculated as NiO, from 5 to 30% by weight of titanium dioxide or zirconium dioxide, from 0 to 20% by weight of alumina, from 20 to 40% by weight of silica and from 0.01 to 1% by weight of an alkali metal oxide, with the proviso that the contents of the individual components in the catalyst sum to 100% by weight.

2. A process as claimed in claim 1, wherein a catalyst as claimed in claim 7 is used.

3. A process as claimed in claim 1, wherein the oligomerization in the reaction zone is carried out at supercritical temperature and supercritical pressure of the olefins used and without the use of additional solvents which are not in the supercritical state in the reaction zone.

4. A process as claimed in claim 1, wherein C₄-olefins are oligomerized and the oligomerization is carried out at from 20 to 280°C and from 10 to 300 bar.

5. A process as claimed in claim 1, wherein C₄-olefins are oligomerized and the oligomerization is carried out at from 10 to 30 bar and from 20 to 140°C.

6. A process as claimed in claim 1, wherein C₄-olefins are oligomerized and the oligomerization is carried out at from 60 to 300 bar and from 160 to 280°C.

7. A catalyst for the oligomerization of olefins of 2 to 6 carbon atoms as claimed in claim 1, consisting essentially of nickel oxide, silica, titanium dioxide or zirconium dioxide and, if required, alumina and containing, after deduction of the loss on ignition following heating at 900°C, from 10 to 70% by weight of nickel oxide, calculated as NiO, from 5 to 30% by weight of titanium dioxide or zirconium dioxide, from 0 to 20% by weight of alumina, from 20 to 40% by weight of silica and from 0.01 to 1% by weight of an alkali metal oxide, with the proviso that the proportions of the individual components sum to 100% by weight, obtainable by precipitating a nickel salt solution which is free of aluminum or contains a dissolved aluminum salt at a pH of from 5 to 9 by the addition of this nickel salt solution to an alkali metal waterglass solution which contains solid titanium dioxide or zirconium dioxide, drying and heating the resulting precipitate at from 350 to 650°C.

8. A catalyst as claimed in claim 7, obtainable by precipitation at from 30 to 90°C.

## Revendications

1. Procédé pour oligomériser des oléfines en C₂ à C₆ non-ramifiées en leurs dimères, trimères et tétramères, à l'aide d'un catalyseur à lit fixe, sous haute pression, à la température ambiante ou à température élevée, caractérisé en ce qu'on utilise un catalyseur qui contient en tant que constituants actifs, après déduction de la perte au feu après recuit à 900°C, 10 à 70 % en poids d'oxyde de nickel, calculés en NiO, 5 à 30 % en poids de dioxyde de titane et/ou de dioxyde de zirconium, 0 à 20 % en poids d'oxyde d'aluminium, 20 à 40 % en poids de dioxyde de silicium et 0,01 à 1 % en poids d'un oxyde d'un métal alcalin, à la condition que la somme des teneurs en les différents constituants du catalyseur soit de 100 % en poids.

2. Procédé selon la revendication 1, caractérisé en ce qu'on utilise un catalyseur selon la revendication 7.

3. Procédé selon la revendication 1, caractérisé en ce qu'on met en oeuvre l'oligomérisation dans la zone de réaction dans des condition supercritiques de température et de pression des oléfines utilisées, et que l'on n'utilise pas de solvants additionnels qui dans la zone de réaction ne se trouvent pas à l'état supercritique.

4. Procédé selon la revendication 1, caractérisé en ce qu'on oligomérise des oléfines en C₄, et que l'on procède à l'oligomérisation à des températures de 20 à 280°C et sous une pression de 10 à 300 bar.

5. Procédé selon la revendication 1, caractérisé en ce qu'on oligomérise des oléfines en C₄ et qu'on procède à l'oligomérisation sous une pression de 10 à 30 bar et à une température de 20 à 140°C.

6. Procédé selon la revendication 1, caractérisé en ce qu'on oligomérise des oléfines en C₄ et qu'on procède à l'oligomérisation sous une pression de 60 à 300 bar et à une température de 160 à 280°C.

7. Catalyseurs pour oligomériser des oléfines ayant de 2 à 6 atomes de carbone selon la revendication 1, constitués pour l'essentiel d'oxyde de nickel, de dioxyde de silicium, de dioxyde de titane et/ou de dioxyde de zirconium, et éventuellement d'oxyde d'aluminium, et contenant, après déduction de la perte au feu après recuit à 900°C, 10 à 70 % en poids d'oxyde de nickel calculés en NiO, 5 à 30 % en poids de dioxyde de titane et/ou de dioxyde de zirconium, 0 à 20 % en poids d'oxyde d'aluminium, 20 à 40 % en poids de dioxyde de silicium et 0,01 à 1 % en poids d'un oxyde d'un métal alcalin, à la condition que la somme des teneurs en les différents constituants soit de 100 % en poids, que l'on peut obtenir par précipitation d'une solution d'un sel de nickel, exempte d'aluminium ou contenant un sel d'aluminium dissous, à un pH de 5 à 9, par addition de cette solution d'un sel de nickel à une solution d'un orthosilicate d'un métal alcalin, qui contient du dioxyde de titane et/ou du dioxyde de zirconium solide, séchage et recuit du précipité obtenu, à une température de 350 à 650°C.

8. Catalyseurs selon la revendication 7, pouvant être obtenus par précipitation à une température de 30 à 90°C.
